# EUROPÄISCHE PATENTSCHRIFT

(11) **EP 2 921 118 B1**
(45) Veröffentlichungstag und Bekanntmachung des Hinweises auf die Patenterteilung: **20.12.2017**
(21) Anmeldenummer: 15000497.6
(22) Anmeldetag: 20.02.2015
(51) Int. Cl.: A61B 17/16

(54) **Werkzeug zum Erzeugen eines Hinterschnitts in einem Knochen**
Tool for producing an indentation in a bone
Outil de fabrication d'une contre-dépouille dans un os

(30) Priorität: 18.03.2014 DE 102014003721
(43) Veröffentlichungstag der Anmeldung: 23.09.2015
(73) Patentinhaber: Karl Storz GmbH & Co. KG, 78532 Tuttlingen (DE)
(72) Erfinder: Berberich, Sascha, 78532 Tuttlingen (DE)
(74) Vertreter: Fugmann, Winfried

(56) Entgegenhaltungen:
- WO-A1-2014/089198
- DE-A1-102006 007 232
- US-A1- 2013 138 110

## Beschreibung

Die vorliegende Erfindung betrifft ein Werkzeug zum Erzeugen eines Hinterschnitts in einem menschlichen oder tierischen Knochen, insbesondere zum Erzeugen eines Hinterschnitts in einer in den Knochen eingebrachten Bohrung zum Befestigen eines Knochenankers.

Um Weichgewebe, beispielsweise ein Band oder eine Sehne, an einem Knochen zu befestigen, ist es bekannt, in den Knochen eine Bohrung, insbesondere in Form eines Sacklochs, einzubringen und in die Bohrung einen Knochenanker einzusetzen. Der Knochenanker verbleibt in dem Knochen und gibt einem Faden, einem Band oder einem anderen Verbindungsmittel, mit dem das Weichgewebe an dem Knochen befestigt wird, einen festen Halt. Hierdurch kann beispielsweise bei einer Verletzung ruptiertes Weichteilgewebe an der Inzisionsstelle fixiert werden. Derartige bekannte, als Implantate ausgebildete Knochenanker nutzen häufig eine Presspassung oder große Gewindeflanken, um im Knochen einen sicheren Halt zu erlangen. Insbesondere bei Press-Fit-Implantaten, etwa einem Schlaganker mit Rippen oder bei Interferenzschrauben, ist der Außendurchmesser des Implantats größer als der Bohrungsdurchmesser. Dabei wird zum Erlangen des sicheren Halts von innen eine Kraft auf die Wand der Bohrung ausgeübt, die im Extremfall zu einer Fraktur des Knochens führen kann.

Es ist auch bekannt, in einer in einen Knochen eingebrachten Bohrung, insbesondere in einem Sackloch; einen Hinterschnitt zu erzeugen und dadurch eine Kavität zu schaffen, in der ein Knochenanker formschlüssig gehalten werden kann. In US 5,928,239 ist eine chirurgische Vorrichtung zur Erzeugung einer Kavität in einem Knochen offenbart, die einen langerstreckten Schaft und eine Schneidspitze aufweist, die mit einem frei drehbaren Gelenk an der Spitze des Schafts angeordnet ist. Durch Drehung des Schafts mit einer ausreichenden Geschwindigkeit wird die Schneidspitze in eine abgewinkelte Position ausgelenkt und dadurch eine Kavität erzeugt, deren Radius durch die Länge der Schneidspitze bestimmt ist. Aus EP 2 098 177 A1 ist ein chirurgisches Instrument mit einem Schaft bekannt, bei dem am distalen Ende des Schafts ein Schneidelement befestigt ist, das aus einer mit der Längsachse des Schafts fluchtenden Position in eine ausgelenkte Position gebracht werden kann. Gemäß WO 2007/047065 A1 weist ein chirurgisches Instrument zur Erzeugung einer Kavität am distalen Ende eines Schafts ein seitliches Fenster auf, wobei eine Schneide, Hacke oder Spitze von einer Position innerhalb des Schafts in eine Position, die zumindest teilweise aus dem Fenster herausragt, gebracht werden kann. Die genannten bekannten Werkzeuge sind jedoch relativ komplex aufgebaut und sind hinsichtlich ihrer Handhabung bei der Anwendung, insbesondere bei kleinen Bohrungsdurchmessern, nicht optimal.

In der nicht vorveröffentlichten internationalen Patentanmeldung WO 2014/089198 A1 ist ein kanülierter retrograder Bohrlochräumer mit einer Bohrkrone, einem langerstreckten äußeren Rohrschaft und einem oder mehreren Schneidelementen offenbart. Durch Verschieben des äußeren Rohrschafts in Richtung auf die Bohrkrone treffen die Schneidelemente auf einen Anschlag der Bohrkrone und rotieren nach außen. Der Bohrlochräumer kann mit einem Führungsdraht verwendet werden.

Es ist Aufgabe der vorliegenden Erfindung, ein Werkzeug zum Erzeugen eines Hinterschnitts in einem Knochen anzugeben, das einfach aufgebaut ist und/oder hinsichtlich der Handhabung zur einfachen, raschen und risikoarmen Erzeugung einer Kavität mit einem Hinterschnitt in einem Knochen verbessert ist.

Diese Aufgabe wird durch ein Werkzeug gemäß Anspruch 1 gelöst.

Vorteilhafte Weiterbildungen der Erfindung ergeben sich aus den Unteransprüchen.

Ein erfindungsgemäßes Werkzeug zum Erzeugen eines Hinterschnitts in einem Knochen eines Menschen oder eines Tiers, insbesondere in einer in den Knochen eingebrachten Sacklochbohrung, umfasst einen Kern, der einen langerstreckten Schaft und einen am distalen, d.h. benutzerfernen, Ende des Schafts angesetzten Stempel umfasst. Der Schaft weist einen geringeren Außendurchmesser als der Stempel auf. Der Schaft und der Stempel können massiv oder hohl ausgebildet sein. Der Stempel ist vorzugsweise fest mit dem Schaft verbunden. Der Stempel weist in einem Übergangsbereich zum Schaft eine in distaler Richtung ansteigende Anlaufschräge auf. Die Anlaufschräge kann insbesondere eine sich in distaler Richtung verbreiternde, bezüglich einer Längsachse des Kerns rotationssymmetrische Anlauffläche sein. Der Stempel kann im Wesentlichen zylindrisch ausgebildet sein, wobei im proximalen, d.h. benutzernäheren, Endbereich des Stempels die Mantelfläche des Stempels über die Anlaufschräge bzw. die Anlauffläche in die Mantelfläche des ebenfalls im Wesentlichen zylindrischen Schafts übergeht. Dabei erleichtert die zylindrische Ausbildung des Stempels die Einführung des Stempels in die zuvor angelegte Bohrung in dem Knochen sowie die Führung und Zentrierung in der Bohrung. Vorzugsweise ist der Stempel distalseitig geschlossen und distal mit einer randseitigen Fase versehen, um eine atraumatische Einführung in die Bohrung zu ermöglichen. Insbesondere kann der Stempel distalseitig stumpf ausgebildet sein, wobei das distale Ende des Stempels vorzugsweise durch eine konvex gewölbte oder eine senkrecht zu einer Längsachse des Schafts stehende ebene Fläche gebildet wird, die sich im Wesentlichen über die gesamte Breite des Stempels erstreckt und eine randseitige Fase aufweisen kann.

Das erfindungsgemäße Werkzeug umfasst weiterhin eine auf dem Schaft des Kerns längsverschiebbar angeordnete Schneidhülse. Die Schneidhülse ist im Wesentlichen rohrförmig ausgebildet und umschließt den Schaft des Kerns zumindest abschnittsweise. Das distale Ende der Schneidhülse ist als mindestens ein Segment ausgebildet, das derart abwinkelbar mit einem Schaft der Schneidhülse verbunden ist, dass es durch Aufschieben auf die Anlaufschräge seitlich abspreizbar ist. Das Segment ist somit in einer ersten Position, die es einnimmt, wenn es die Anlaufschräge nicht berührt, innerhalb einer Verlängerung der Au-ßenkontur des Schafts der Schneidhülse angeordnet und wird durch Aufschieben auf die Anlaufschräge in eine zweite Position abgewinkelt, in der es über die Außenkontur der Schneidhülse hinausragt. Insbesondere kann die Schneidhülse mit dem mindestens einen Segment einstückig ausgebildet sein. Vorzugsweise fluchtet die Außenkontur des Stempels mit der Außenkontur der Schneidhülse, so dass das Werkzeug bei auf den Schaft des Kerns aufgeschobener Schneidhülse, jedoch noch nicht abgespreiztem Segment, im Wesentlichen eine zylindrische Form hat. In diesem Zustand kann das Werkzeug leicht in eine in einem Knochen angelegte Bohrung, die einen dem Außendurchmesser des Stempels und der Schneidhülse entsprechenden oder größeren Innendurchmesser aufweist, eingeführt werden; es kann auch vorgesehen sein, dass zunächst der Stempel in die Bohrung eingeführt und sodann die Schneidhülse nachgeführt wird.

Das mindestens eine Segment weist eine seitlich angeordnete Schneidkante auf. Die Schneidkante, die auch als Schneide eines Schneidmessers ausgebildet sein kann, kann durch Rotation der Schneidhülse um ihre Längsachse in eine tangentiale Richtung, zu der die Schneidkante senkrecht steht oder schräg gerichtet ist, bewegt werden. Die Schneidkante kann dabei in Gewebe, das das Werkzeug umfangsseitig umgibt, einschneiden. Insbesondere kann auf diese Weise Knochenmaterial, das die Wand der Bohrung bildet, in die das Werkzeug eingeführt worden ist, abgetragen werden. Das mindestens eine Segment kann auch zum Abtragen von Gewebe durch Bewegung in axialer Richtung ausgebildet sein und hierfür beispielsweise an seiner Stirnseite ebenfalls eine Schneidkante bzw. ein Schneidmesser aufweisen. Die Schneidhülse kann somit auch als Bohrhülse ausgebildet sein. Die Schneidhülse und/oder der Kern können für eine einmalige oder eine mehrfache Verwendung ausgelegt sein.

Dadurch, dass das Werkzeug einen Kern mit einem Stempel umfasst, der eine proximalseitig angeformte Anlauffläche aufweist, und dass durch Aufschieben auf die Anlauffläche ein distalseitiges Segment der Schneidhülse, das eine seitliche Schneidkante aufweist, abgespreizt werden kann, wird ein Abtragen von Knochengewebe in seitlicher Richtung in einer in einen Knochen eingebrachten Bohrung durch Rotation der Schneidhülse ermöglicht. Durch Aufschieben auf die Anlauffläche und Rotation der Schneidhülse kann in einem Sackloch eine Kavität mit einem Hinterschnitt geschaffen werden, in die ein Knochenanker formschlüssig einsetzbar ist. Dabei ist die Tiefe der Hinterschneidung bzw. der Durchmesser der Kavität durch den Betrag des Aufschiebens des Segments auf die Anlaufschräge kontrollierbar, während die Ausdehnung der Kavität in Längsrichtung des Werkzeugs durch eine gemeinsame Längsbewegung von Kern und Schneidhülse bestimmt werden kann. Hierdurch wird ein einfach aufgebautes und sicher und einfach, insbesondere ohne hohe Kraftaufwendung, bedienbares Werkzeug zum Erzeugen einer Hinterschneidung in einer in einen Knochen eingebrachten Bohrung geschaffen.

Gemäß einer bevorzugten Ausführungsform der Erfindung ist das distale Ende der Schneidhülse als eine Mehrzahl von Segmenten ausgebildet, die durch distalseitig in das distale Ende der Schneidhülse eingebrachte Schlitze gebildet werden. Mindestens eines der Segmente, vorzugsweise alle Segmente, weisen jeweils mindestens eine seitlich angeordnete Schneidkante auf. Durch das Einbringen von Schlitzen in das distale Ende der Schneidhülse können auf einfache Weise mehrere Segmente mit seitlich angeordneten Kanten erzeugt werden, die jeweils als Schneidkanten dienen können. Die Schneidkanten können durch den Rand der Schlitze gebildet werden, der scharfkantig sein kann, oder können Schneiden von am Rand der Schlitze angeordneten Schneidmessern sein. Das bzw. die Segmente können auch beidseitig Schneidkanten aufweisen, um eine Schneidwirkung bei einer Rotation in beiden Drehrichtungen zu ermöglichen. Durch die mehreren nebeneinander angeordneten Segmente kann durch eine Rotation der Schneidhülse um ihre Längsachse Knochengewebe besonders wirksam abgetragen werden. Ferner wird eine besonders einfache Herstellung der Schneidhülse durch Einbringen von Schlitzen in das distale Ende eines Rohres ermöglicht.

Die Schlitze bzw. die durch die Schlitze gebildeten Schneidkanten sowie gegebenenfalls an den Schneidkanten angeordnete Schneidmesser können parallel oder schräg zur Längsachse gerichtet sein. Insbesondere bei einer schrägen, etwa einer schraubenförmigen Ausbildung der Schneidkanten bzw. Schlitze kann eine verbesserte Schneidwirkung erzielt sowie der Abtransport von abgeschnittenen Gewebeteilen erleichtert werden.

Vorzugsweise ist die mindestens eine Schneidkante des mindestens einen Segments gekrümmt und/oder gezahnt; sofern die Schneidwirkung durch ein an einer Kante des mindestens einen Segments angeordnetes Schneidmesser erzielt wird, gilt dies für das bzw. die Schneidmesser. Eine gekrümmte Schneidkante kann kontinuierlich gekrümmt sein, so dass sich in einem Längsschnitt eine Rundung ergibt, oder auch eine nach außen gerichtete Ecke oder Spitze aufweisen. Hierdurch ist eine weiter verbesserte gewebeabtragende Wirkung erzielbar.

In vorteilhafter Weise kann das mindestens eine Segment mit Bezug auf eine vorgegebene Rotationsrichtung der Schneidhülse eine führende und eine folgende Kante aufweisen, wobei die führende Kante zumindest teilweise als Schneidkante ausgebildet ist; die folgende Kante muss nicht als Schneidkante ausgebildet sein. Insbesondere kann es vorgesehen sein, dass die folgende Kante in Bezug auf die Längsachse der Schneidhülse tiefer liegt als die führende Kante. Die folgende Kante ist somit bei einem kleineren Radius als die führende Kante angeordnet. Eine die führende mit der folgenden Kante verbindende Außenfläche des mindestens einen Segments verläuft dementsprechend geneigt zu einer tangentialen Richtung. Hierdurch wird es ermöglicht, dass die führende Kante besonderes wirksam in das seitlich angeordnete Gewebe einschneidet und zugleich ein verbesserter Abtransport von abgeschnittenen Gewebespänen erfolgt.

Vorzugsweise ist ein Übergangsbereich zwischen dem mindestens einen Segment und dem Schaft der Schneidhülse geschwächt. Dies kann beispielsweise durch Materialausdünnung, etwa durch eine in den Übergangsbereich innen- oder außenseitig eingebrachte Nut oder Kerbe, oder durch Verwendung eines anderen Materials an der Basis der Segmente erreicht werden, so dass eine geringere Kraft notwendig ist, um das mindestens eine Segment beim Aufschieben auf die Anlaufschräge im Übergangsbereich zu biegen und dadurch abzuspreizen. Hierdurch werden das Abspreizen und damit die Bedienung des Werkzeugs weiter erleichtert sowie die Haltbarkeit eines für mehrfache Verwendung ausgelegten Werkzeugs verbessert.

Erfindungsgemäß besteht die Schneidhülse, zumindest der Übergangsbereich zwischen dem Segment und dem Schaft der Schneidhülse, aus einem elastischen Material. Ein solches Material kann beispielsweise Federstahl, Nitinol oder ein anderes elastisches metallisches oder auch nichtmetallisches Material sein. Das Segment selbst und/oder der Schaft der Schneidhülse können ebenfalls das elastische Material aufweisen oder aus diesem bestehen. Nach einer Abspreizung des mindestens einen Segments durch Abbiegen im Übergangsbereich wird dieses bei Zurückziehen der Schneidhülse von der Anlaufschräge durch die von dem elastischen Material erzeugte Rückstellkraft wieder in seine ursprüngliche, innerhalb der Verlängerung der Außenkontur des Schafts der Schneidhülse liegende Position zurückgeführt. Hierdurch wird das Zurückziehen des Werkzeugs nach erfolgter Einbringung des Hinterschnitts in den Knochen erleichtert und das den Hinterschnitt bildende Knochenmaterial geschont.

Gemäß einer bevorzugten Ausführungsform der Erfindung ist eine distalseitige innere Kante der Schneidhülse bzw. des mindestens einen Segments abgerundet oder weist eine Fase auf. Hierdurch wird die Reibung beim Aufschieben auf die Anlaufschräge vermindert und die Bedienbarkeit des Werkzeugs erleichtert. Zusätzlich kann auch eine seitliche innere Kante des mindestens einen Segments abgerundet sein oder eine Fase aufweisen; dies ist insbesondere in dem Fall vorteilhaft, dass bei der Rotation der Schneidhülse zum Abtragen von Gewebe der Kern nicht rotiert.

Vorzugsweise ist die Anlaufschräge näherungsweise konisch, insbesondere glockenförmig ausgebildet. Eine konisch geformte Anlauffläche ermöglicht eine besonders wirksame gleichzeitige Abspreizung aller Segmente, die das distale Ende der Schneidhülse bilden, beim Aufschieben der Schneidhülse auf die Anlaufschräge. Die Anlaufschräge kann insbesondere ein im Längsschnitt gekrümmtes Profil aufweisen, vorzugsweise ein solches, das nach distal mit zunehmender Steigung ansteigt. Hierdurch wird ein Abspreizen des mindestens einen Segments mit einer gleichmäßigen oder einer mit zunehmender Abspreizung zunehmenden Kraft ermöglicht und dadurch die Handhabung des Werkzeugs erleichtert. Insbesondere ermöglicht eine glockenförmig ausgebildete Anlauffläche eine besonders leicht steuerbare Abspreizung einer Mehrzahl von Segmenten am distalen Ende der Schneidhülse.

Gemäß einer bevorzugten Ausführungsform der Erfindung weist der Schaft des Kerns oder auch ein Übergangsbereich zwischen dem Stempel und dem Schaft des Kerns einen in axialer Richtung wirkenden Anschlag auf, der mit einem entsprechenden Anschlag der Schneidhülse zum Begrenzen eines axialen Verschiebewegs der Schneidhülse relativ zum Kern zusammenwirkt. Insbesondere können der Anschlag des Kerns und der der Schneidhülse jeweils als Stufe ausgebildet sein, die ein Verschieben der Schneidhülse relativ zum Kern bis zu einem vorgegebenen maximalen Verschiebeweg erlaubt, bei dem eine Abspreizung des mindestens einen Segments einem vorgegebenen Durchmesser der zu erzeugenden Kavität entspricht. Hierdurch wird die Erzeugung eines für eine bestimmte Anwendung bzw. für ein bestimmtes Implantat genau passenden Hinterschnitts erleichtert.

Weiterhin ist es bevorzugt, dass zwischen dem Schaft des Kerns und der Schneidhülse ein axial durchgehender Zwischenraum angeordnet ist, durch den eine Spülflüssigkeit geleitet werden kann. Hierdurch wird eine Spülung zum Abtransport von abgeschnittenen Knochenspänen oder anderen Gewebeteilen sowie eine Kühlung zur Vermeidung einer Überhitzung des Werkzeugs oder des umgebenen Knochengewebes beim Betrieb ermöglicht. Um eine sichere Führung der Schneidhülse auf dem Schaft des Kerns zu ermöglichen, können an der Außenseite des Schafts des Kerns und/oder an der Innenseite des Schafts der Schneidhülse Führungsmittel, etwa Führungsrippen, angeordnet sein, die in Längsrichtung verlaufen und zwischen denen der axial durchgehende Zwischenraum zur Durchleitung von Spülflüssigkeit verbleibt.

In vorteilhafter Weise können der Schaft des Kerns mit einem axial durchgehenden Hohlraum und der Stempel zumindest abschnittsweise hohl ausgebildet sein, wobei ein innerhalb des Stempels gebildeter Hohlraum mit dem durchgehenden Hohlraum des Schafts des Kerns verbunden ist und wobei der Stempel seitliche Durchbrüche aufweist. Die Durchbrüche können in einem zylindrischen Abschnitt des Stempels und/oder im Bereich der Anlaufschräge bzw. in der Anlauffläche angeordnet sein. Hierdurch wird der Durchfluss von Spülflüssigkeit zum Abtransport von Knochenspänen oder anderen Gewebeteilen sowie zur Kühlung ermöglicht.

Gemäß einer besonders bevorzugten Ausführungsform sind sowohl ein axial durchgehender Zwischenraum zwischen dem Schaft des Kerns und der Schneidhülse als auch ein bis in den Stempel durchgehender Hohlraum innerhalb des Kerns vorgesehen, wobei der Hohlraum des Kerns über Durchbrüche im Stempel mit dem Außenraum des Stempels in Verbindung steht. Hierdurch werden ein kontinuierlicher Durchfluss von Spülflüssigkeit und ein besonders effektiver Abtransport abgeschnittener Gewebeteile sowie eine besonders wirksame Kühlung ermöglicht. So kann beispielsweise durch den Zwischenraum zwischen dem Schaft des Kerns und der Schneidhülse Spülflüssigkeit zugeführt werden und durch die Durchbrüche im Stempel, den Hohlraum innerhalb des Stempels und den Hohlraum innerhalb des Schafts des Kerns eine Absaugung von Spülflüssigkeit erfolgen. Hierdurch wird die Verwendbarkeit des erfindungsgemäßen Werkzeugs weiter verbessert. An einem distalen Ende des Werkzeugs können ein oder mehrere Anschlüsse zur Verbindung mit einer Spül- und Saugeinrichtung angeordnet sein.

Bei einem nicht beanspruchten Verfahren zum Erzeugen eines Hinterschnitts in einem menschlichen oder tierischen Knochen wird ein wie oben beschrieben ausgebildetes Werkzeug verwendet. Das Verfahren geht aus von einem Bohrkanal, der in an sich bekannter Weise zuvor in dem Knochen angelegt worden ist, insbesondere von einem Sackloch.

Zur Durchführung des Verfahrens wird eine Schneidhülse auf den Schaft eines Kerns aufgeschoben, wobei die Schneidhülse und der Kern wie oben beschrieben ausgebildet sind. Die Schneidhülse wird so weit auf dem Schaft des Kerns vorgeschoben, dass das mindestens eine Segment noch nicht über die Anlaufschräge aufgespreizt wird. Der Kern und die auf diesen aufgesetzte Schneidhülse werden in das Bohrloch eingeführt und gemeinsam im Bohrloch vorgeschoben, bis der distale Endbereich der Schneidhülse in einem Bereich des Bohrlochs angeordnet ist, in dem eine Kavität mit einem Hinterschnitt angelegt werden soll; insbesondere kann der Stempel des Kerns bis zum Boden des Sacklochs vorgeschoben werden. Alternativ kann zunächst der Kern in das Bohrloch eingeführt und danach die Schneidhülse über den Schaft des Kerns nachgeführt werden. Es ist auch möglich, dass ein Bohrer, mit dem die Bohrung erzeugt worden ist, als Kern ausgebildet ist und nach Einbringen der Bohrung in dem Bohrloch verbleibt und zum Erzeugen des Hinterschnitts die Schneidhülse über den Schaft des Kerns vorgeschoben wird.

Die Schneidhülse wird spätestens jetzt in Rotation versetzt und weiter nach distal vorgeschoben, so dass beim Auftreffen auf die Anlaufschräge das mindestens eine Segment abgespreizt wird und dabei um die Längsachse der Schneidhülse rotiert. Durch die Rotationsbewegung trägt die Schneidkante des mindestens einen Segments Knochengewebe an der Wand des Sacklochs ab, wobei der Durchmesser einer dadurch erzeugten Kavität durch das Maß des Aufschiebens der Schneidhülse auf die Anlaufschräge des Kerns bestimmt ist. Durch gemeinsames axiales Bewegen des Kerns und der auf die Anlaufschräge aufgeschobenen Schneidhülse wird die Länge der mit Hilfe des Hinterschnitts erzeugten Kavität bestimmt. Insbesondere können der Kern und die Schneidhülse gemeinsam unter Rotation zurückgezogen werden, um ausgehend vom Boden des Sacklochs oder, je nach Länge des Stempels, nahe dem Boden des Sacklochs, eine Hinterschneidung zu erzeugen.

Nachdem eine Kavität mit den gewünschten Dimensionen erzeugt worden ist, wird, gegebenenfalls nach geringfügigem Vorschieben des Kerns, die Schneidhülse zurückgezogen, so dass das mindestens eine Segment zurückfedert oder durch Berührung mit dem Knochenmaterial zurückgedrückt wird und wieder innerhalb der Verlängerung der Außenkontur der Schneidhülse zu liegen kommt. Die Schneidhülse und der Kern werden danach aus dem Bohrloch entnommen.

Zum Erzielen einer Rotationsbewegung kann die Schneidhülse mit einem mechanischen oder elektrischen Antrieb verbindbar sein, der etwa nach Art des Antriebs eines chirurgischen Bohrers oder Morcellators ausgebildet sein kann. Ferner kann eine Verbindung zu einer externen Spül- und Saugeinrichtung hergestellt werden, um eine Spülung durch einen zwischen dem Schaft des Kerns und der Schneidhülse angeordneten Hohlraum sowie durch Durchbrüche in der Wandung des Stempels und einen in diesem und im Schaft des Kerns anschließenden Hohlraum erfolgen. Hierdurch können Gewebespäne, die bei der Erzeugung des Hinterschnitts entstehen, entfernt werden; gleichzeitig kann eine übermäßige Erwärmung des Werkzeugs und des knocheninneren Raums vermieden werden. Die Verbindung mit dem Antrieb sowie mit der Spül- und Saugeinrichtung wird vorzugsweise vor dem Einschieben der Schneidhülse in das Bohrloch hergestellt, kann aber auch danach bewerkstelligt werden.

In dem auf diese Weise erzeugten knocheninneren Hohlraum kann sodann ein Implantat, etwa ein Knochenanker, durch das Bohrloch eingeführt und durch Spreizung in der Kavität formschlüssig, vorzugsweise formfüllend, verankert werden. Ein hierfür geeigneter Knochenanker ist beispielsweise aus US 2010/0331881 A1 bekannt.

Vorzugsweise steht beim Erzeugen des Hinterschnitts der Kern still, d.h. der Schaft des Kerns wird von einem Bohrantrieb nicht angetrieben. Die Schneidhülse rotiert somit auf dem Kern, und das mindestens eine Segment rotiert auf der insbesondere konisch oder glockenförmig ausgebildeten Anlauffläche des Stempels. In diesem Fall ist in besonders bevorzugter Weise die Innenseite des mindestens einen Segments abgerundet ausgebildet, insbesondere sind die seitlichen inneren Kanten des mindestens einen Segments abgerundet oder weisen eine Fase auf, um die Reibung an der Anlauffläche des Stempels zu verringern. Da der in das Sackloch eingeführte Stempel bei dieser Verfahrensvariante stillsteht, ist hierbei eine besonders sichere Führung der Schneidhülse gewährleistet; ferner kann eine Überwärmung von Knochengewebe im Bereich des Bodens des Sacklochs besonders sicher vermieden werden.

Alternativ kann beim Erzeugen des Hinterschnitts der Kern gemeinsam mit der Schneidhülse rotieren. In diesem Fall kann die Entstehung von Abrieb zwischen dem abgespreiztem mindestens einen Segment und der Anlaufschräge des Stempels vermieden werden; ferner kann der Antrieb besonders einfach ausgebildet sein. Die Anlaufschräge kann in diesem Fall an die Anzahl und Anordnung der abspreizbaren Segmente angepasst sein und beispielsweise in dem Fall, dass nur ein einziges Segment vorgesehen ist, nur einseitig ausgebildet sein. Durch Spülung kann ebenfalls eine Überwärmung des am Rand der Kavität verbleibenden Knochengewebes vermieden werden.

Weitere Aspekte der Erfindung ergeben sich aus der nachfolgenden Beschreibung eines bevorzugten Ausführungsbeispiels und der beigefügten Zeichnung. Es zeigen:
Fig. 1a bis 1c ein erstes Ausführungsbeispiel eines erfindungsgemäßen Werkzeugs in drei unterschiedlichen axialen Relativpositionen von Schneidhülse und Kern;
Fig. 2 ein zweites Ausführungsbeispiel eines erfindungsgemäßen Werkzeugs;
Fig. 3a und 3b das Werkzeug gemäß Fig. 2 in Schnittansichten in zwei unterschiedlichen axialen Relativpositionen und Fig. 3c ein vergrößertes Detail aus Fig. 3b;
Fig. 4a und 4b ein drittes Ausführungsbeispiel eines erfindungsgemäßen Werkzeugs mit der Schneidhülse in einer Schrägansicht (Fig. 4a) sowie das Werkzeug mit der Schneidhülse in einer Schnittansicht (Fig. 4b);
Fig. 5 ein viertes Ausführungsbeispiel eines erfindungsgemäßen Werkzeugs in einer Schnittansicht;
Fig. 6 ein fünftes Ausführungsbeispiel eines erfindungsgemäßen Werkzeugs in einer Schnittansicht;
Fig. 7 die Schneidhülse gemäß einem sechsten Ausführungsbeispiel eines erfindungsgemäßen Werkzeugs in einer Schrägansicht;
Fig. 8a und 8b ein siebtes Ausführungsbeispiel eines erfindungsgemäßen Werkzeugs in Schnittansichten in zwei unterschiedlichen axialen Relativpositionen;
Fig. 9 den Kern gemäß einem achten Ausführungsbeispiel der Erfindung;
Fig. 10 den Kern gemäß einem neunten Ausführungsbeispiel der Erfindung;
Fig. 11 ein weiteres Ausführungsbeispiel eines erfindungsgemäßen Werkzeugs in einer Schnittansicht;
Fig. 12 einen Schnitt durch einen Knochen mit einer gemäß einem nicht beanspruchten Verfahren geschaffenen Kavität mit einem in diese eingesetzten Knochenanker;
Fig. 13 einen Schnitt durch einen Knochen mit einer gemäß einer Variante des Verfahrens geschaffenen Kavität.

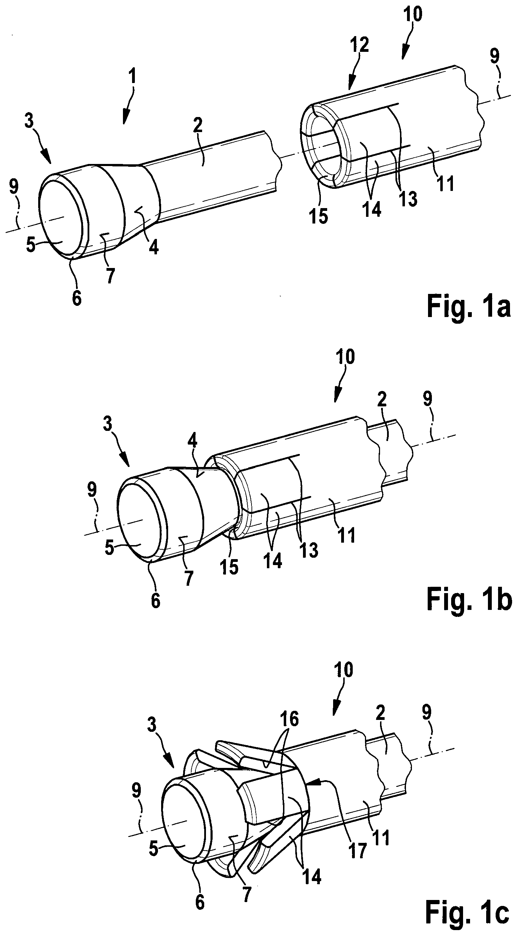

Ein erfindungsgemäßes Werkzeug besteht gemäß einer ersten Ausfuhrungsform, die in Fig. 1a gezeigt ist, aus einem Kern 1 und einer Schneidhülse 10, wobei der Kern 1 aus einem zylindrischen Schaft 2 und einem an dessen distalem Ende ansetzenden Stempel 3 besteht. Der Stempel 3 ist im Wesentlichen ebenfalls zylindrisch ausgebildet und hat einen größeren Außendurchmesser als der Schaft 2. In seinem proximalen Endbereich weist der Stempel eine im Wesentlichen kegelförmige Anlauffläche 4 auf, deren Durchmesser sich vom Durchmesser der Schafts 2 nach distal auf dem Außendurchmesser des Stempels 3 erweitert. Distalseitig ist der Stempel 3 mit einer Stirnwand 5 abgeschlossen, die randseitig mit einer Fase 6 in die Mantelfläche 7 des Stempels 3 übergeht.

Wie in Fig. la gezeigt, umfasst die Schneidhülse 10 einen zylindrischen Schaft 11, dessen distaler Endbereich 12 durch eine Mehrzahl von sich in Längsrichtung erstreckenden Schlitzen 13 in eine Mehrzahl von Segmenten 14 geteilt ist. Die Segmente weisen an ihrer distalen Innenseite eine Fase oder eine Abrundung 15 auf. Der Innendurchmesser des Schafts 11 der Schneidhülse 10 ist derart auf den Außendurchmesser des Schafts 2 des Kerns 1 abgestimmt, dass die Schneidhülse auf dem Schaft 2 des Kerns 1 längsverschiebbar ist, insbesondere aus proximaler Richtung über den Schaft 2 geschoben werden kann. Der Außendurchmesser der Schneidhülse 10 stimmt mit dem des Stempels 3 überein. Zur Verdeutlichung ist die Wandstärke der Schneidhülse 10 in Fig. 1a und den weiteren Figuren im Verhältnis zum Durchmesser größer dargestellt, als bei den beschriebenen Ausführungsbeispielen bevorzugt ist. Am proximalen Ende der Schneidhülse 10 bzw. des Kerns 2 können eine Handhabe oder eine Kupplung zur Verbindung mit einem Antrieb vorgesehen sein (nicht dargestellt).

Fig. 1b zeigt die Schneidhülse 10 in einer Position, in der sie so weit nach distal über den Schaft 2 des Kerns geschoben ist, dass die Segmente 14 die Anlauffläche 4 berühren oder nahezu berühren, aber noch nicht aufgespreizt werden. Wie in Fig. 1a bis 1c angedeutet, sind die Schneidhülse 10 und der Kern 1 nach dem Aufschieben koaxial mit einer gemeinsamen Längsachse 9 angeordnet. Wird nun die Schneidhülse 10 weiter in distaler Richtung vorgeschoben, so werden die Segmente 14 durch Aufschieben auf die Anlauffläche 4 gespreizt und stehen dadurch über die Außenkontur der Anordnung aus Kern 1 und Schneidhülse 10 vor (Fig. 1c). Ein Übergangsbereich 17 zwischen den Segmenten 14 und dem Schaft 11 der Schneidhülse wirkt dabei als Gelenk und ist hierfür elastisch biegbar ausgebildet. Das Aufschieben und Aufspreizen wird durch die Abrundung 15 an der distalen inneren Kante der Segmente 14 erleichtert. Jedes Segment 14 weist an einer seitlichen Au-ßenkante eine Schneidkante 16 auf. Durch Aufspreizen der Segmente 14 und Rotation der Schneidhülse 10 um ihre Längsachse 9 können die Schneidkanten 16 in umgebendes Gewebe einschneiden und dieses abtragen.

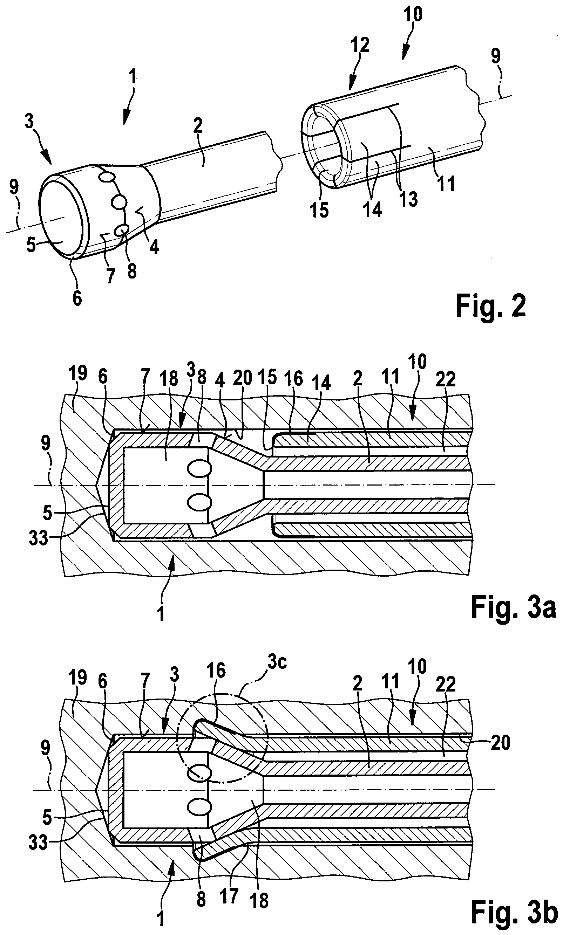

Gemäß der in Fig. 2 dargestellten zweiten Ausführungsform der Erfindung können der Schaft 2 und der Stempel 3 jeweils einen inneren Hohlraum aufweisen, wobei der Hohlraum des Schafts 2 mit dem des Stempels 3 verbunden ist und bis zum proximalen Ende des Schafts 2 zum Anschluss an eine Absaugung durchgehend ausgeführt ist (nicht dargestellt). Die Mantelfläche 7 des Stempels 3 weist eine Anzahl von bis in die Anlauffläche 4 reichenden Durchbrüchen 8 auf, durch die der Hohlraum des Stempels 3 mit dem Außenraum in Verbindung steht. Der Innendurchmesser der Schneidhülse des Schafts 11 der Schneidhülse 10 ist größer als der Außendurchmesser des Schafts 2 des Kerns 1, so dass durch den hierdurch gebildeten Zwischenraum Spülflüssigkeit in den Bereich der Anlauffläche 4 geführt werden kann. Am proximalen Ende der Schneidhülse 10 kann hierfür ein Spülanschluss vorgesehen sein (nicht dargestellt). Auf diese Weise kann ein geschlossener Spülkreislauf zum Abtransport von Knochengewebe, das mit den Schneidkanten 16 abgetragen worden ist, geschaffen werden. Im nicht dargestellten proximalen Endbereich des Schafts 2 des Kerns 1 und der Schneidhülse 10 sind Spül- und Sauganschlüsse sowie eine Handhabe oder eine Kupplung zum Verbinden mit einem Antrieb angeordnet.

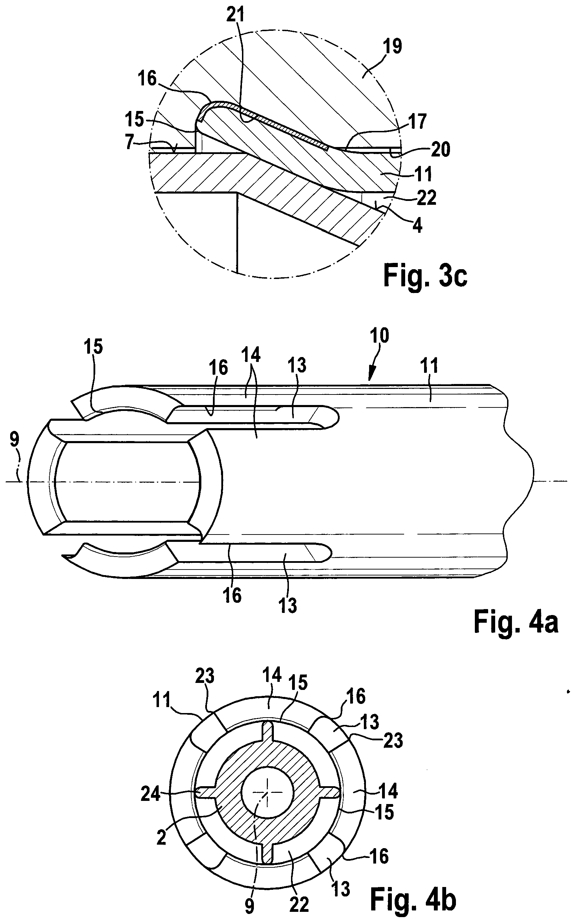

In Fig. 3a und 3b ist das Werkzeug gemäß Fig. 2 in zwei Relativpositionen, die den für die erste Ausführungsform in Fig. 1b und 1c gezeigten Situationen entsprechen, jeweils in einer in Längsrichtung geschnittenen Schnittansicht dargestellt; Fig. 3c zeigt ein vergrößertes Detail. Bei der in Fig. 3a gezeigten Position der Schneidhülse 10 liegen die Segmente 14 innerhalb der durch die Außenseite des Schafts 11 der Schneidhülse 10 sowie durch die Mantelfläche 7 des Stempels 3 definierten Außenkontur. Durch Aufschieben auf die Anlauffläche 4 werden die Segmente 14 gespreizt und ragen dann über die Außenkontur vor (Fig. 3b). Wie in Fig. 3c vergrößert dargestellt, ist die distale innere Kante der Segmente 14 mit einer Abrundung 15 versehen, um Reibung und Verschleiß beim Gleiten auf der Anlauffläche 4 zu verringern. Ferner ist auf mindestens einer Seite eines jeden Segments 14 eine durch die Ausführung des Schlitzes 13 entstandene Kante als Schneidkante 16 ausgebildet. Die Schneidkante 16 ist in einem Längsprofil gerundet ausgebildet und erstreckt sich in proximaler Richtung bis in den Übergangsbereich 17 zwischen dem betreffenden Segment 14 und dem Schaft 11 der Schneidhülse 10 oder nahe an diesen heran, um das Herausarbeiten des Hinterschnitts 21 zu erleichtern. Um auch bei einer Verschiebung in distaler Richtung Gewebe abtragen zu können, erstreckt sich die Schneidkante 16 bis an oder in die Abrundung 15 hinein.

Wie in den Figuren 3a bis 3c gezeigt, sind der Kern 1, ein distaler Abschnitt des Schafts 2 des Kerns 1, die Segmente 14 und ein distaler Abschnitt des Schafts 11 der Schneidhülse 10 in eine in einen Knochen 19 eingebrachte Bohrung 20, die als Sackloch mit einem kegelförmigen Boden 33 ausgebildet ist, eingeführt. Der Außendurchmesser des Stempels 3 und der Schneidhülse 10 ist geringfügig kleiner als der Innendurchmesser der Bohrung 20, um das Einführen in die Bohrung 20 zu erleichtern. Ferner wird hierdurch die Spülung erleichtert. Zur Spülung wird Spülflüssigkeit durch einen zwischen dem Schaft 2 des Kerns 1 und dem Schaft 11 der Schneidhülse 10 verlaufenden durchgehenden Zwischenraum 22 zugeführt, passiert den Arbeitsbereich der Schneidkanten 16, gelangt durch die Durchbrüche 8 in den im Inneren des Stempels 3 und des Schafts 2 des Kerns angeordneten Hohlraum 18 und wird durch diesen nach proximal abgeführt. Im nicht dargestellten proximalen Endbereich des Schafts 2 des Kerns 1 und der Schneidhülse 10 sind Spül- und Sauganschlüsse sowie eine Kupplung zum Verbinden mit einem Antrieb angeordnet.

Zum Erzeugen eines Hinterschnitts 21 in der Bohrung 20 werden der Kern 1 und die Schneidhülse 10 gemeinsam oder nacheinander soweit in die Bohrung 20 eingeführt, bis der Stempel 3 mit seiner Stirnwand 5 am Boden des Sacklochs anliegt (Fig. 3a). Sodann wird die Schneidhülse 10 in Rotation um die Längsachse 9 versetzt und relativ zum Kern 1 weiter in distaler Richtung verschoben, um die Segmente 14 aufzuspreizen und einen Hinterschnitt 21 in die Seitenwand der Bohrung 20 einzubringen (Fig. 3b und 3c).

In Fig. 4a und 4b ist ein drittes Ausführungsbeispiel der Erfindung in zwei unterschiedlichen Ansichten gezeigt, wobei in Fig. 4b die auf den Schaft 2 des Kerns 1 aufgeschobene Schneidhülse 10 im Bereich der Segmente 14 quer zur Längsachse geschnitten ist. Dabei sind die in die Schneidhülse 10 distalseitig eingebrachten Schlitze 13 breit ausgeführt, um genügend Raum für die Durchströmung mit Spülflüssigkeit zu schaffen. Ferner ist, wie in Fig. 4a und 4b zu erkennen ist, die Schneidkante 16 als in tangentialer Richtung vorspringende Schneide an einer Seitenwand des Schlitzes 13 ausgebildet. Hierdurch wird ein besonders effektives Abtragen von Knochenmaterial ermöglicht. Dabei ist eine Rotationsrichtung beim Betreiben des Werkzeugs vorgegeben, wobei die Schneidkante 16 an der führenden Kante des Segments 14 angeordnet ist. Die folgende Kante 23 weist keine Schneide auf. Ferner trägt der Schaft 2 der Kerns 1 an seiner Außenseite eine Mehrzahl in Längsrichtung verlaufender Rippen 24, die den Zwischenraum 22 in in Längsrichtung verlaufende Hohlräume unterteilen, durch die Spülflüssigkeit in den Bereich der Schneidkanten 16 geführt werden kann und die zudem eine sichere Führung des Schafts 11 der Schneidhülse 10 auf dem Schaft 2 des Kerns 1 gewährleisten. Im Übrigen sind die Schneidhülse 10 und der Kern 1 wie zuvor beschrieben ausgebildet.

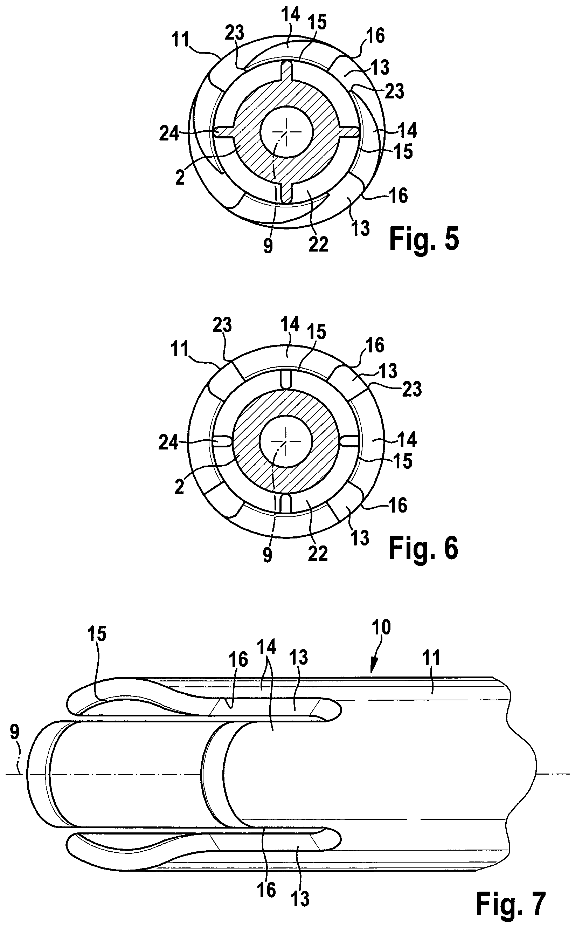

Wie in Fig. 5 in einer der Fig. 4b entsprechenden Darstellung eines vierten Ausführungsbeispiels der Erfindung gezeigt, können die äußeren Seiten der Segmente 14 schräg verlaufen, so dass die führende Kante, die als Schneidkante 16 ausgebildet ist, erhöht gegenüber der folgenden Kante 23 ist. Hierdurch werden die Schneidwirkung und der Abtransport der erzeugten Knochenspäne weiter verbessert. Im Übrigen entspricht das vierte Ausführungsbeispiel dem in Fig. 4a und 4b dargestellten dritten Ausführungsbeispiel.

In Fig. 6 ist in einer der Fig. 4b entsprechenden Darstellung angedeutet, dass die Rippen 24 an der Innenseite des Schafts 11 der Schneidhülse 10 angeordnet sein können. In gleicher Weise wie zu Fig. 4b erläutert wird hierdurch der Zwischenraum 22 in axial verlaufende Hohlräume zur Durchleitung von Spülfluid unterteilt; ferner dienen die Rippen 24 zur sicheren Führung der Schneidhülse 10 auf dem Schaft 2.

Wie in Fig. 7 gezeigt, können die Segmente 14 gemäß einer weiteren Ausführungsform der Erfindung distalseitig abgerundet sein. Auch die Schneidkanten 16, die hier entlang der Außenseite der Segmente 14 verlaufen, sind daher gekrümmt und erstrecken sich mindestens bis zum Scheitelpunkt des jeweiligen Segments 14. Eine derart ausgebildete Schneidhülse 10 ermöglicht ein besonders feinfühliges Abtragen von Knochengewebe.

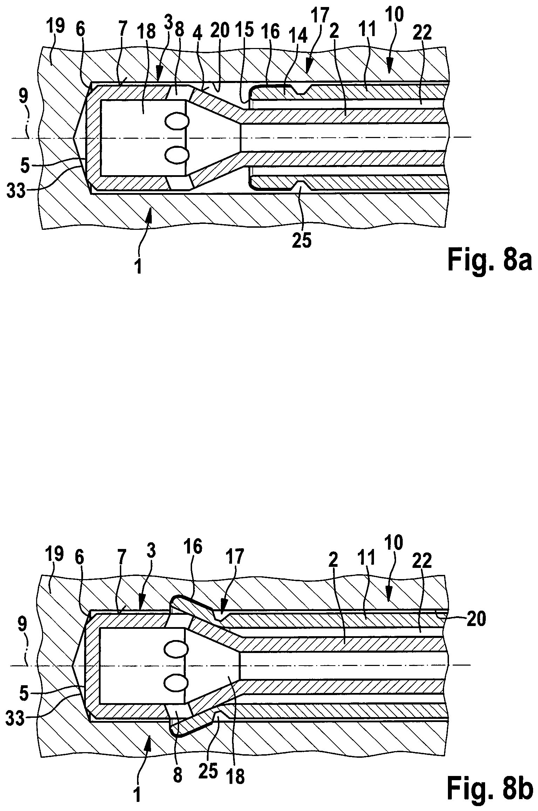

Eine Schneidhülse 10 gemäß den zuvor beschriebenen Ausführungsbeispielen kann ferner derart ausgebildet sein, das im Übergangsbereich 17 zwischen den Segmenten 14 und dem Schaft 11 der Schneidhülse 10 die Wandstärke der Schneidhülse 10 verringert ist, um eine leichtere Abwinkelung des Segments 14 zu ermöglichen. Hierfür kann beispielsweise eine Nut oder eine Kerbe 25 im Übergangsbereich 17 eingebracht sein, wie dies beispielhaft in den Figuren 8a und 8b in zwei verschiedenen axialen Relativpositionen von Kern 1 und Schneidhülse 10, die den in Fig. 3a und 3b gezeigten entsprechen, dargestellt ist. Im Übrigen entsprechen die in den Figuren 6 bis 8b dargestellten Ausführungsformen der Erfindung den zuvor erläuterten.

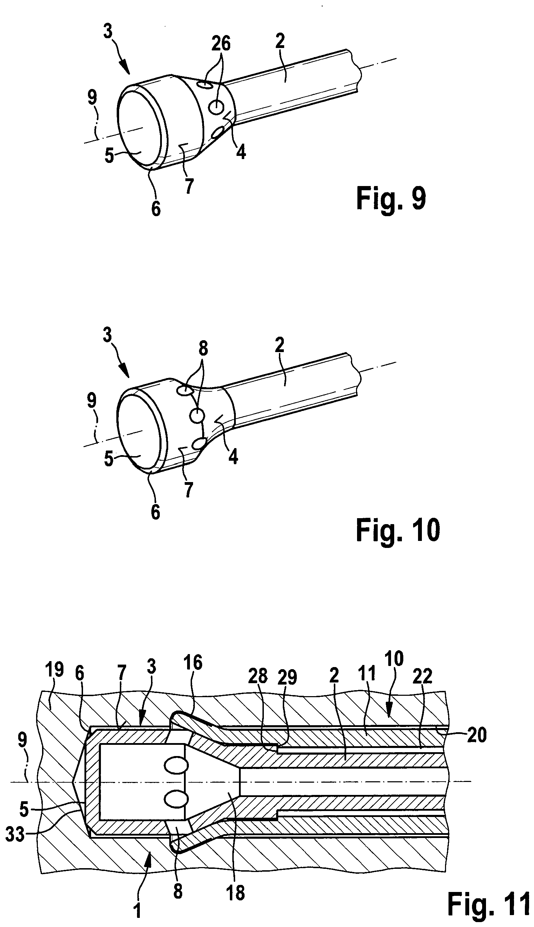

In Fig. 9 ist der Stempel 3 eines Kerns 1 gemäß einer achten Ausführungsform der Erfindung gezeigt. Hierbei ist der Stempel 3 ebenfalls am distalen Ende eines Schafts 2 angeordnet und geht mit einer konischen Anlauffläche 4 in die Mantelfläche des Schafts 2 über. Der zylindrische Bereich des Stempels 3 weist keine Durchbrüche auf, jedoch weist die Anlauffläche 4 Durchbrüche 26 auf, die länglich ausgebildet sein können und die eine Verbindung vom Außenraum in den inneren Hohlraum des Stempels 3 bilden, der mit dem durchgehenden Hohlraum des Schafts 2 verbunden ist. Zur Verminderung von Reibung durch die Rotation der an der Anlauffläche 4 anliegenden Segmente können die Durchbrüche 26 abgerundete Ränder aufweisen.

Eine weitere Ausführungsform eines Stempels 3 ist in Fig. 10 dargestellt. Hierbei ist die Anlauffläche 4 glockenförmig ausgebildet, wobei die Steigung der hierdurch gebildeten Anlaufschräge von proximal nach distal zunimmt. Im Übrigen ist der Stempel 3 bei den in den Figuren 9 und 10 gezeigten Ausführungsbeispielen wie zuvor beschrieben ausgebildet und kann in Kombination mit den zuvor beschriebenen Schneidhülsen 10 verwendet werden. Auch bei den in Fig. 2a bis 3c sowie Fig. 10 dargestellten Ausführungsbeispielen kann die Anlauffläche 4 einen oder mehrere Durchbrüche 26 aufweisen, um die Spülung insbesondere zu Beginn der Herausarbeitung des Hinterschnitts zu verbessern.

Wie in Fig. 11 gezeigt, kann in einem distalen Endbereich des Schafts 2 eine Stufe 28 vorgesehen sein, die mit einer Stufe 29 an der Innenseite des Schafts 11 der Schneidhülse 10 zusammenwirkt, um den Verschiebeweg in axialer Richtung zu begrenzen. Hierdurch kann ein Außendurchmesser einer zu erzeugenden Kavität besonders genau festgelegt werden. Im Übrigen ist das in Fig. 11 gezeigte Ausführungsbeispiel wie die zuvor beschriebenen Ausführungsbeispiele ausgebildet.

Aus der Beschreibung der Ausführungsbeispiele ergeben sich vielfältige Kombinationsmöglichkeiten eines Stempels 3 und eines Schafts 2 sowie eines durch den Stempel 3 und den Schaft 2 gebildeten Kerns 1 mit einer Schneidhülse 10, die ebenfalls von der Erfindung umfasst sind. Der Stempel 3 gemäß den zuvor beschriebenen Ausführungsbeispielen kann insbesondere einen Außendurchmesser von etwa 2 bis 5 mm aufweisen. Der Außendurchmesser der Schneidhülse 10 beträgt ebenfalls ca. 2 bis 5 mm, wobei die Schneidhülse 10 und die Segmente 14 eine Wandstärke von beispielsweise ca. 0,2 mm haben können. Die Schneidhülse 10 besteht aus einem für die chirurgische Anwendung geeigneten elastischen Material, vorzugsweise aus einem elastischen metallischen Material, etwa Federstahl oder Nitinol. Derartige Materialien ermöglichen ein mehrfaches Abbiegen und Zurückfedern der Segmente 14 sowie die Ausbildung von Schneidkanten 16, die auch hartes Knochenmaterial abtragen können. Der Stempel 3 und der Schaft 2 des Kerns 1 bestehen aus einem für die chirurgische Anwendung geeigneten metallischen oder nichtmetallischen Material, etwa aus Edelstahl oder Polyetheretherketon (PEEK). Der Kern 1 kann einstückig ausgebildet sein.

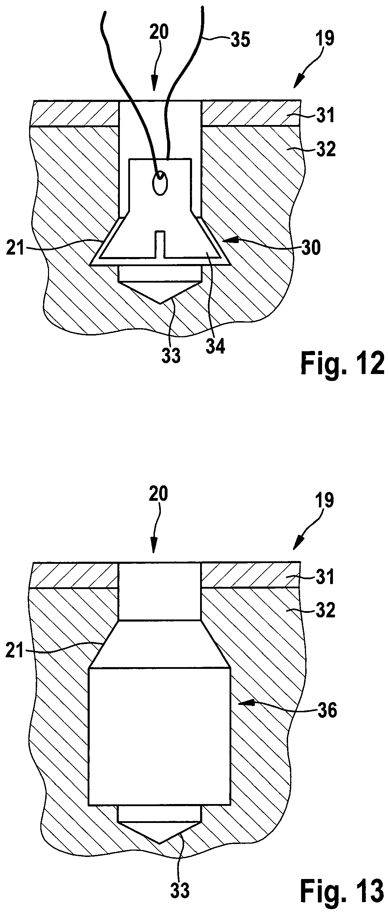

In Fig. 12 ist schematisch ein Beispiel für eine Kavität 30 dargestellt, die mit einem erfindungsgemäßen Werkzeug geschaffen werden kann. In den Knochen 19 wird zunächst mit einem üblichen chirurgischen Knochenbohrer eine zylindrische Bohrung 20 eingebracht. Die Bohrung 20 durchdringt die äußere Schicht (Kortikalis 31) und endet in der Spongiosa 32 des Knochens 19. Der Boden 33 der Bohrung 20 ist, entsprechend der Form des verwendeten Bohrers, in der Regel kegelförmig ausgebildet. Nach dem Einbringen der Bohrung 20 wird der Bohrer aus dieser wieder herausgezogen.

Nun wird der Stempel 3 des Kerns 1 in die Bohrung 20 eingeführt, bis dieser am Boden 33 der Bohrung anliegt. Sodann wird die auf den Schaft 2 des Kerns 1 aufgeschobene Schneidhülse 10 in die Bohrung 20 eingeführt (s. Fig. 1b, 3a, 8a). Die Schneidhülse 10 wird nach Berührung der Anlauffläche 4 unter Rotation um ihre Längsachse noch weiter in distaler Richtung geschoben, wodurch die Segmente 14 aufgespreizt werden und in das umgebende Knochengewebe (Spongiosa 32) einschneiden (s. Fig. 1c, 3b, 3c, 8b). Hierdurch entsteht ein Hinterschnitt 21. Alternativ können der Kern 1 und die Schneidhülse 10 in der in Fig. 1b, 3a und 8a gezeigten Relativposition gemeinsam in die Bohrung 20 eingeschoben und nach Anliegen des Stempels 3 am Boden 33 der Bohrung 20 die Schneidhülse weiter in distaler Richtung bewegt werden. Vorzugsweise wird der Kern 1 nicht in Rotation versetzt, dies kann aber auch möglich sein.

Wenn ein Hinterschnitt 21 ausreichender Tiefe erzeugt worden ist, wird zunächst die Schneidhülse 10 in proximaler Richtung zurückgezogen. Die Segmente 14 liegen nach Herabgleiten von der Anlauffläche 4 wieder innerhalb der Verlängerung der Außenkontur des Schafts 11 der Schneidhülse 10 (s. Fig. 1b, 3a, 8a), so dass die Schneidhülse 10 ohne weiteres Abtragen von Knochengewebe aus der Bohrung 20 entnommen werden kann. Danach wird auch der Kern 1 aus der Bohrung 20 herausgezogen. In die Kavität 30, die durch den Hinterschnitt 21 gebildet wird, wird sodann ein Knochenanker 34 eingeführt, mit einem Spreizmechanismus gespreizt und dadurch in der Kavität 30 verankert. Der Knochenanker 34 ermöglicht somit, etwa über einen Faden 35 Weichgewebe oder auch ein chirurgisches Implantat an der Außenseite des Knochens 19 zu halten.

Mit einem erfindungsgemäßen Werkzeug kann auch eine wie in Fig. 13 geformte, im Wesentlichen zylindrische Kavität 36 in dem Knochen 19 geschaffen werden. Dabei wird zunächst wie zu Fig. 12 beschrieben vorgegangen und, nachdem die Segmente 14 aufgespreizt worden sind, werden unter weiterer Rotation der Schneidhülse 10 der Kern 1 und die Schneidhülse 10 gemeinsam in proximaler Richtung verschoben. Wenn eine Kavität 36 mit ausreichender Länge entstanden ist, wird der Kern 1 festgehalten oder geringfügig in distaler Richtung bewegt und die

Schneidhülse 10 weiter in proximaler Richtung aus der Bohrung 20 herausgezogen. Dann wird der Kern 1 ebenfalls aus der Bohrung 20 entnommen.

Durch das zu den Figuren 12 und 13 beschriebene Verfahren kann eine Kavität 30, 36 zum formschlüssigen Fixieren eines Knochenankers mit für viele Anwendungen geeigneten Dimensionen geschaffen werden. In der Regel reicht dabei ein Hinterschnitt von ca. 0,5 mm Tiefe aus, d.h. die Kavität 30, 36 ist im Durchmesser gegenüber der Bohrung um ca. 1 mm erweitert. Der Knochenanker kann beispielsweise eine Länge von ca. 10 mm haben.

Der Übersichtlichkeit halber sind nicht in allen Figuren alle Bezugszeichen dargestellt. Zu einer Figur nicht erläuterte Bezugszeichen haben die gleiche Bedeutung wie in den übrigen Figuren.

### Bezugszeichenliste

- 1: Kern
- 2: Schaft
- 3: Stempel
- 4: Anlauffläche
- 5: Stirnwand
- 6: Fase
- 7: Mantelfläche
- 8: Durchbruch
- 9: Längsachse
- 10: Schneidhülse
- 11: Schaft
- 12: Distaler Endbereich
- 13: Schlitz
- 14: Segment
- 15: Abrundung
- 16: Schneidkante
- 17: Übergangsbereich
- 18: Hohlraum
- 19: Knochen
- 20: Bohrung
- 21: Hinterschnitt
- 22: Zwischenraum
- 23: Folgende Kante
- 24: Rippe
- 25: Kerbe
- 26: Durchbruch
- 28: Stufe
- 29: Stufe
- 30: Kavität
- 31: Kortikalis
- 32: Spongiosa
- 33: Boden
- 34: Knochenanker
- 35: Faden
- 36: Kavität

## Patentansprüche

1. Werkzeug zum Erzeugen eines Hinterschnitts (21) in einem Knochen (19), umfassend einen Kern (1) mit einem Schaft (2) und einem an einem distalen Ende des Schafts (2) angesetzten Stempel (3), wobei der Stempel (3) in einem Übergangsbereich zum Schaft (2) eine in distaler Richtung ansteigende Anlaufschräge aufweist, und eine auf dem Schaft (2) längsverschiebbar angeordnete Schneidhülse (10), deren distales Ende durch mindestens ein durch Aufschieben auf die Anlaufschräge abspreizbares Segment (14) gebildet wird, wobei das mindestens eine Segment (14) mindestens eine seitlich angeordnete Schneidkante (16) aufweist und wobei die Schneidhülse (10) zumindest in einem Übergangsbereich zwischen dem mindestens einen Segment (14) und einem Schaft (11) der Schneidhülse (10) aus einem elastischen Material besteht.

2. Werkzeug nach Anspruch 1, **dadurch gekennzeichnet, dass** das distale Ende der Schneidhülse (10) durch eine Mehrzahl von jeweils mindestens eine seitlich angeordnete Schneidkante (16) aufweisenden Segmenten (14) gebildet wird, die durch distalseitig in das distale Ende der Schneidhülse (10) eingebrachte Schlitze (13) voneinander getrennt sind.

3. Werkzeug nach Anspruch 1 oder 2, **dadurch gekennzeichnet, dass** die Schneidkante bzw. Schneidkanten (16) schräg zu einer Längsachse (9) der Schneidhülse (10) angeordnet ist bzw. sind.

4. Werkzeug nach einem der vorhergehenden Ansprüche, **dadurch gekennzeichnet, dass** die Schneidkante bzw. Schneidkanten (16) gekrümmt und/oder gezahnt ist bzw. sind.

5. Werkzeug nach einem der vorhergehenden Ansprüche, **dadurch gekennzeichnet, dass** das mindestens eine Segment (14) mit Bezug auf eine vorgegebene Rotationsrichtung der Schneidhülse (10) eine führende Kante und eine folgende Kante (23) aufweist, wobei die führende Kante zumindest teilweise als Schneidkante (16) ausgebildet ist und die folgende Kante (23) radial tiefer als die führende Kante angeordnet ist.

6. Werkzeug nach einem der vorhergehenden Ansprüche, **dadurch gekennzeichnet, dass** ein Übergangsbereich zwischen dem mindestens einen Segment (14) und einem Schaft (11) der Schneidhülse (10) geschwächt ist.

7. Werkzeug nach einem der vorhergehenden Ansprüche, **dadurch gekennzeichnet, dass** eine distale innere Kante des mindestens einen Segments (14) eine Abrundung (15) oder eine Fase aufweist.

8. Werkzeug nach einem der vorhergehenden Ansprüche, **dadurch gekennzeichnet, dass** die Anlaufschräge als im Wesentlichen konische oder glockenförmige Anlauffläche (4) ausgebildet ist.

9. Werkzeug nach einem der vorhergehenden Ansprüche, **dadurch gekennzeichnet, dass** der Schaft (2) des Kerns (1) oder ein Übergangsbereich zwischen dem Stempel (3) und dem Schaft (2) des Kerns (1) in axialer Richtung einen Anschlag aufweist, der mit einem Anschlag der Schneidhülse (10) zum Begrenzen eines axialen Verschiebewegs der Schneidhülse (10) relativ zum Kern (1) zusammenwirkt.

10. Werkzeug nach einem der vorhergehenden Ansprüche, **dadurch gekennzeichnet, dass** zwischen dem Schaft (2) des Kerns (1) und der Schneidhülse (10) ein axial durchgehender Zwischenraum (22) angeordnet ist.

11. Werkzeug nach einem der vorhergehenden Ansprüche, **dadurch gekennzeichnet, dass** der Stempel (3) und der Schaft (2) des Kerns (1) einen bis in einen proximalen Endbereich des Schafts (2) durchgehenden inneren Hohlraum (18) aufweisen und der Stempel (3) mindestens einen seitlichen Durchbruch (8) aufweist.

## Claims

1. Tool for generating an undercut (21) in a bone (19), comprising a core (1) with a shank (2) and a stamp (3) mounted on a distal end of the shank (2), wherein the stamp (3), in a transition area to the shank (2), has a run-on bevel ascending in the distal direction, and a cutting sleeve (10) which is arranged to be longitudinally movable on the shank (2) and of which the distal end is formed by at least one segment (14) that can be spread open when pushed onto the run-on bevel, wherein the at least one segment (14) has at least one laterally arranged cutting edge (16), and wherein the cutting sleeve (10) is made of an elastic material, at least in a transition area between the at least one segment (14) and a shank (11) of the cutting sleeve (10).

2. Tool according to Claim 1, **characterized in that** the distal end of the cutting sleeve (10) is formed by a plurality of segments (14) which each have at least one laterally arranged cutting edge (16) and which are separated from each other by slits (13) introduced distally into the distal end of the cutting sleeve (10).

3. Tool according to Claim 1 or 2, **characterized in that** the one or more cutting edges (16) are arranged obliquely with respect to a longitudinal axis (9) of the cutting sleeve (10).

4. Tool according to one of the preceding claims, **characterized in that** the one or more cutting edges (16) are curved and/or toothed.

5. Tool according to one of the preceding claims, **characterized in that** the at least one segment (14) has a leading edge and a trailing edge (23) in relation to a predefined rotational direction of the cutting sleeve (10), wherein the leading edge is designed at least partially as cutting edge (16), and the trailing edge (23) is arranged radially deeper than the leading edge.

6. Tool according to one of the preceding claims, **characterized in that** a transition area between the at least one segment (14) and a shank (11) of the cutting sleeve (10) is weakened.

7. Tool according to one of the preceding claims, **characterized in that** a distal inner edge of the at least one segment (14) has a rounding (15) or a chamfer.

8. Tool according to one of the preceding claims, **characterized in that** the run-on bevel is designed as a substantially conical or bell-shaped run-on surface (4).

9. Tool according to one of the preceding claims, **characterized in that** the shank (2) of the core (1) or a transition area between the stamp (3) and the shank (2) of the core (1) has, in the axial direction, an abutment which cooperates with an abutment of the cutting sleeve (10) in order to limit an axial travel of the cutting sleeve (10) relative to the core (1).

10. Tool according to one of the preceding claims, **characterized in that** an axially continuous gap (22) is arranged between the shank (2) of the core (1) and the cutting sleeve (10).

11. Tool according to one of the preceding claims, **characterized in that** the stamp (3) and the shank (2) of the core (1) have an inner hollow space (18) continuing into a proximal end area of the shank (2), and the stamp (3) has at least one lateral aperture (8).

## Revendications

1. Outil de fabrication d'une contre-dépouille (21) dans un os (19), comprenant un noyau (1) avec une tige (2) et un poinçon (3) attaché à une extrémité distale de la tige (2), le poinçon (3) présentant, dans une région de transition à la tige (2), un biseau de montée montant dans la direction distale, et une douille de coupe (10) disposée de manière déplaçable longitudinalement sur la tige (2), dont l'extrémité distale est formée par au moins un segment (14) pouvant être écarté en le poussant sur le biseau de montée, l'au moins un segment (14) présentant au moins une arête de coupe disposée latéralement (16) et la douille de coupe (10), au moins dans une région de transition entre l'au moins un segment (14) et une tige (11) de la douille de coupe (10), se composant d'un matériau élastique.

2. Outil selon la revendication 1, **caractérisé en ce que** l'extrémité distale de la douille de coupe (10) est formée par une pluralité de segments (14) présentant à chaque fois au moins une arête de coupe (16) disposée latéralement, lesquels segments sont séparés les uns des autres par des fentes (13) pratiquées du côté distal dans l'extrémité distale de la douille de coupe (10).

3. Outil selon la revendication 1 ou 2, **caractérisé en ce que** l'arête de coupe ou les arêtes de coupe (16) est/sont disposée (s) obliquement par rapport à un axe longitudinal (9) de la douille de coupe (10) .

4. Outil selon l'une quelconque des revendications précédentes, **caractérisé en ce que** l'arête de coupe ou les arêtes de coupe (16) est/sont courbée(s) et/ou dentée(s).

5. Outil selon l'une quelconque des revendications précédentes, **caractérisé en ce que** l'au moins un segment (14) présente, par rapport à une direction de rotation prédéfinie de la douille de coupe (10), une arête d'attaque et une arête de fuite (23), l'arête d'attaque étant réalisée au moins en partie sous forme d'arête de coupe (16) et l'arête de fuite (23) étant disposée radialement plus profondément que l'arête d'attaque.

6. Outil selon l'une quelconque des revendications précédentes, **caractérisé en en ce qu'**une région de transition entre l'au moins un segment (14) et une tige (11) de la douille de coupe (10) est affaiblie.

7. Outil selon l'une quelconque des revendications précédentes, **caractérisé en ce qu'**une arête distale intérieure de l'au moins un segment (14) présente un arrondi (15) ou un chanfrein.

8. Outil selon l'une quelconque des revendications précédentes, **caractérisé en ce que** le biseau de montée est réalisé sous forme de surface de montée (4) essentiellement conique ou en forme de cloche.

9. Outil selon l'une quelconque des revendications précédentes, **caractérisé en ce que** la tige (2) du noyau (1) ou une région de transition entre le poinçon (3) et la tige (2) du noyau (1) dans la direction axiale présente une butée qui coopère avec une butée de la douille de coupe (10) pour limiter une course de déplacement axiale de la douille de coupe (10) par rapport au noyau (1).

10. Outil selon l'une quelconque des revendications précédentes, **caractérisé en ce qu'**entre la tige (2) du noyau (1) et la douille de coupe (10) est disposé un espace intermédiaire (22) s'étendant axialement.

11. Outil selon l'une quelconque des revendications précédentes, **caractérisé en ce que** le poinçon (3) et la tige (2) du noyau (1) présentent une cavité interne (18) s'étendant en continu jusque dans une région d'extrémité proximale de la tige (2) et le poinçon (3) présente au moins un orifice latéral (8) .
